# EUROPEAN PATENT APPLICATION

(11) **EP 4 202 038 A1**
(43) Date of publication of application: **28.06.2023**
(21) Application number: 21383180.3
(22) Date of filing: 21.12.2021
(51) Int. Cl.: C12N 5/071

(54) **PERICYTES FOR USE AS A MEDICAMENT**

(71) Applicant: Universidad de Murcia, 30072 Murcia (ES)
(72) Inventor: Valdor Alonso, Rut, 30120 Murcia (ES); García Bernal, David, 30120 Murcia (ES); Moraleda Jiménez, José María, 30120 Murcia (ES); Martínez Pérez, Salvador, 30120 Murcia (ES)
(74) Representative: Hoffmann Eitle

(57) **Abstract**

**PERICYTES FOR USE AS A MEDICAMENT.** The present invention refers to a substantially pure population of pericytes, or cell suspension comprising pericytes, wherein the pericytes are at least 80% of the total cell population or suspension, characterized in that CMA is inhibited, for example by inhibiting the expression or deleting the gene LAMP2A. In a preferred embodiment, said population or suspension of pericytes is used as a medicament, for instance in the treatment of cancer, preferably glioblastoma.

## Description

### FIELD OF THE INVENTION

The present invention refers to the medical filed. Particularly, the present invention refers to an isolated pericyte cell, the secretome derived thereof, a substantially pure population of pericytes, or cell suspension comprising pericytes, wherein the pericyte is characterized in that the chaperone-mediated autophagy (CMA) is inhibited or impaired, for instance by inhibiting the expression or deleting the gene LAMP2A. In a preferred embodiment, said population or suspension of pericytes is used as a medicament, for example in the treatment of cancer, preferably glioblastoma (GB).

### STATE OF THE ART

GB is considered by the U.S. National Cancer Institute as the most aggressive form of brain cancer. GB represents 15.4% of all primary brain tumors and about 60%-75% of all astrocytomas and shows rapid growth rate of malignant cells in the organ, with a survival rate of 14-15 months. There is no curative treatment available, and new therapies and prognostic factors are unmet needs for GB.

During GB development, the tumor cells infiltrate and invade the cerebral parenchyma interacting with the cells of the perivascular areas and establishing a functional network. PC are perivascular stromal cells with stem cell like-properties that can phagocyte and promote an immune defence in response to brain damage. PC acquire an immunosuppressive function during the progression of GB that contributes to the establishment of immunotolerance and, therefore, to tumor growth. This immune function depends on the aberrant upregulation of GB-induced chaperone-mediated autophagy (CMA) through cell-cell interactions, that causes PC to present an anti-inflammatory phenotype and inactivate the T-cell responses for tumor removal. Therefore, as CMA is universal in all types of cells, including tumor cells, a better understanding of the biology of these cells that surround the tumor is needed to find target specific markers and establish an effective selective therapy.

The present invention is focused on solving the above cite problem and, departing from a depth analysis of PC biology and CMA understanding, a novel therapeutic strategy is herein proposed for the treatment of cancer, preferably GB.

### DESCRIPTION OF THE INVENTION

### Brief description of the invention

As explained above, the present invention refers to an isolated pericyte cell, the secretome derived thereof, a substantially pure population of pericytes, or cell suspension comprising pericytes, wherein the pericyte is characterized in that the chaperone-mediated autophagy (CMA) is inhibited or impaired, for instance by inhibiting the expression or deleting the gene LAMP2A. In a preferred embodiment, said population or suspension of PC is used as a medicament, for example in the treatment of cancer, preferably GB.

So, modified PC (characterized in that CMA is inhibited or impaired, for instance by inhibiting the expression or deleting the gene LAMP2A) are used in the context of the present invention as an anti-tumor immunogenic mechanism which is effective in the elimination of tumor cells, mainly GB.

Particularly, the inventors of the present invention propose that CMA, which is a protein selective degradation mechanism, represents a novel target for the local control of both PC-tumor cell interaction, and PC modulation of the anti-tumor immune response that facilitates tumor growth. Furthermore, the identification of new markers related to GB-induced CMA in PC might be essential for the diagnosis and prognosis of the GB cancer. Indeed, the inventors of the present invention propose that interference with the immunosuppressive function of PC by different strategies through CMA blocking besides reducing tumor growth, represents a novel target for the development of new therapies against GB and other tumors arising in microvascularized tissues containing PC.

Remarkably, the inventors of the present invention have found several gene expression pathways differentially enriched in LAMP2A-KO PC and affected by GB-induced CMA in PC. Bioinformatic analyses showed that the phagosome formation, cellular senescence, focal adhesion and the effector function to promote anti-tumor immune responses were the most affected pathways, revealing a transcriptomic profiling of specific target functions useful for future therapy. In addition, several molecules associated to pro-tumoral or anti-tumoral functions such as gelsolin, periostin, osteopontin, lumican and vitamin D, were identified in the secretome of pro-tumoral GB-conditioned PC or anti-tumoral deficient CMA PC in presence of GB. The CMA ablation in PC co-cultured with GB cells showed an immunogenic phenotype able to phagocyte GB cells and a key strategy to develop therapies against GB tumor progression. A novel intravenous therapy using exofucosylated CMA-deficient PC was efficient to make PC reach the tumoral niche and facilitate tumor elimination.

Thus, the results provided by the present invention shows the impaired immunogenic function of PC with GB-induced CMA, driving to other altered PC functions and the identifications of new target markers for GB prognosis/therapy. The present invention shows CMA ablation in PC as a key target mechanism to develop a successful therapy against GB tumor progression.

On the other hand, it is important to note that therapy with unmodified control PC promotes an immunosuppressive environment. PC are cells with immune function that have mesenchymal cell properties and can promote a regenerative and anti-inflammatory environment optimal for the treatment of diseases where cell proliferation and regeneration are needed. Therefore, therapy with unmodified PC, which are also capable of reaching the inflammatory niche, is of great importance for the treatment of inflammatory and degenerative diseases.

So, in summary, according to the present invention, modified PC (characterized in that CMA is inhibited or impaired, for example by inhibiting the expression or deleting the gene LAMP2A) can be used as a cellular cancer therapy, for example against GB. On the other hand, non-modified PC can favour mesenchymal and anti-inflammatory properties and consequently they can be used in the treatment of inflammatory and degenerative diseases.

So, the first embodiment of the present invention refers to an isolated PC cell (hereinafter *"PC of the invention"*), or secretome derived thereof, wherein the PC is characterized in that the CMA is inhibited or impaired.

In a preferred embodiment, the PC of the invention is characterized in that the expression of the gene LAMP2A is inhibited or the gene LAMP2A is deleted.

In a preferred embodiment, the PC of the invention derives from adipose tissue or any vascular-stromal compartment of microvascularized tissues, comprising brain, breast, kidney or liver.

The second embodiment of the present invention refers to a substantially pure population of the PC of the invention or cell suspension comprising the PC of the invention (*hereinafter "substantially pure population or cell suspension of the invention"*), wherein the PC are at least 80% of the total cell population or suspension.

The third embodiment of the present invention refers to the PC of the invention, or to the substantially pure population or cell suspension of the invention, for use as a medicament, preferably in the treatment of cancer, more preferably in the treatment of GB. Alternatively, this embodiment refers to a method for the treatment of cancer, more preferably for the treatment of GB, which comprises administering to the patient a therapeutically effective dose or amount of the PC of the invention, or of the substantially pure population or cell suspension of the invention.

In a preferred embodiment the CMA of the pericyte is inhibited or impaired before being administered to the patient.

The fourth embodiment of the present invention refers to a pharmaceutical composition comprising the PC of the invention, or the substantially pure population or cell suspension of the invention, and, optionally, pharmaceutically acceptable carriers or excipients.

The fifth embodiment of the present invention refers to an *in vitro* method for obtaining the PC of the invention, or the substantially pure population or cell suspension of the invention, for use as a medicament, wherein the method comprises inhibiting CMA in the PC, preferably by deleting the gene LAMP2A or inhibiting its expression.

In a preferred embodiment the present invention refers to an *in vitro* method for obtaining the PC of the invention, or the substantially pure population or cell suspension of the invention, for use in the treatment of cancer, preferably glioblastoma, wherein the method comprises inhibiting CMA in the PC, preferably by deleting the gene LAMP2A or inhibiting its expression.

In a preferred embodiment the gene LAMP2A is deleted using a gene editing technique selected from: CRISPR-Cas Gene Editing Restriction Enzymes, Zinc Finger Nucleases, TALENs Gene Editing or RNA interference.

In a preferred embodiment the method comprises inhibiting CMA, preferably inhibiting the expression of the gene LAMP2A, using pharmaceutical or biologic inhibitors.

The sixth embodiment of the present invention refers to the in *vitro* use of the PC of the invention, the secretome derived thereof, or the substantially pure population or suspension of the invention for obtaining biomarkers to be used in the diagnosis of cancer, preferably glioblastoma.

The seventh embodiment of the present invention refers to an *in vitro* method for the diagnosis and/or prognosis of cancer, preferably for the diagnosis of glioblastoma, which comprises assessing, in the secretome of a pericyte characterized in that the CMA is inhibited or impaired, isolated from a subject, the level of at least a biomarker selected from the group comprising: lumican, vitamin D, gelsolin, periostin and/or osteopontin, wherein:
a. The identification of a higher level of lumican and/or vitamin D as compared with a pre-established threshold level determined in healthy control subjects, is an indication that the patient suffering from cancer, preferably from glioblastoma, has a good prognosis, and/or a good chance of therapy success.
b. The identification of a higher level of gelsolin, periostin and osteopontin as compared with a pre-established threshold level determined in healthy control subjects, is an indication that the patient suffering from cancer, preferably from glioblastoma, has poor prognosis.

Other the other hand, the present invention also includes the following embodiments:
Particularly, the eight embodiment of the present invention refers to a pharmaceutical composition comprising a substantially pure population of non-modified or natural PC, or cell suspension comprising non-modified or natural PC, wherein the PC are at least 80% of the total cell population or suspension and, optionally, pharmaceutically acceptable excipient or carrier.

The ninth embodiment of the present invention refers to a substantially pure population of non-modified or natural PC, or cell suspension comprising non-modified or natural PC, wherein the PC are at least 80% of the total cell population or suspension, for use as a medicament, preferably in the treatment of inflammatory and/or degenerative diseases. Alternatively, this embodiment refers to a method for the treatment of inflammatory and/or degenerative diseases, which comprises administering to the patient a therapeutically effective dose or amount of a substantially pure population of non-modified or natural PC, or cell suspension comprising non-modified or natural PC.

For the purpose of the present invention, the following terms are defined:
- The term "comprising" means including, but not limited to, whatever follows the word "comprising". Thus, the use of the term "comprising" indicates that the listed elements are required or mandatory but that other elements are optional and may or may not be present.
- The term "consisting of' means including, and limited to, whatever follows the phrase "consisting of'. Thus, the phrase "consisting of' indicates that the listed elements are required or mandatory and that no other elements may be present.
- "Pharmaceutically acceptable excipient or carrier" refers to an excipient that may optionally be included in the compositions of the invention and that causes no significant adverse toxicological effects to the patient.
- By "therapeutically effective dose or amount" of the pharmaceutical composition of the invention is intended an amount that, when administered to the subject, brings about a positive therapeutic response in a subject suffering from cancer, preferably GB. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.
- The term "pre-established threshold level" typically refers to the level measured in healthy patients. The patient is likely to suffer from cancer with a given sensitivity and specificity if the levels of the biomarker in the patient are above said "pre-established threshold level". A "threshold value" or "cut-off value" can be determined experimentally, empirically, or theoretically. A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Preferably, the person skilled in the art may compare the level of the biomarker obtained according to the method of the invention with a defined "threshold value". In one embodiment of the present invention, the "threshold value" is derived from the level of biomarker determined in a control sample derived from one or more subjects who are substantially healthy. In one embodiment of the present invention, the "threshold value" may also be derived from the level of the biomarker determined in a control sample derived from one or more subjects who suffers from cancer. Furthermore, retrospective measurement of the level of the biomarker in properly banked historical subject samples may be used in establishing these "threshold values". Typically, the optimal sensitivity and specificity (and so the "threshold value") can be determined using a Receiver Operating Characteristic (ROC) curve based on experimental data. For example, after determining the levels of the biomarker in a group of reference, one can use algorithmic analysis for the statistic treatment of the measured concentrations of biomarkers in biological samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-speciftcity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is quite high. This algorithmic method is preferably done with a computer. Existing software or systems in the art may be used for the drawing of the ROC curve.

### Description of the figures

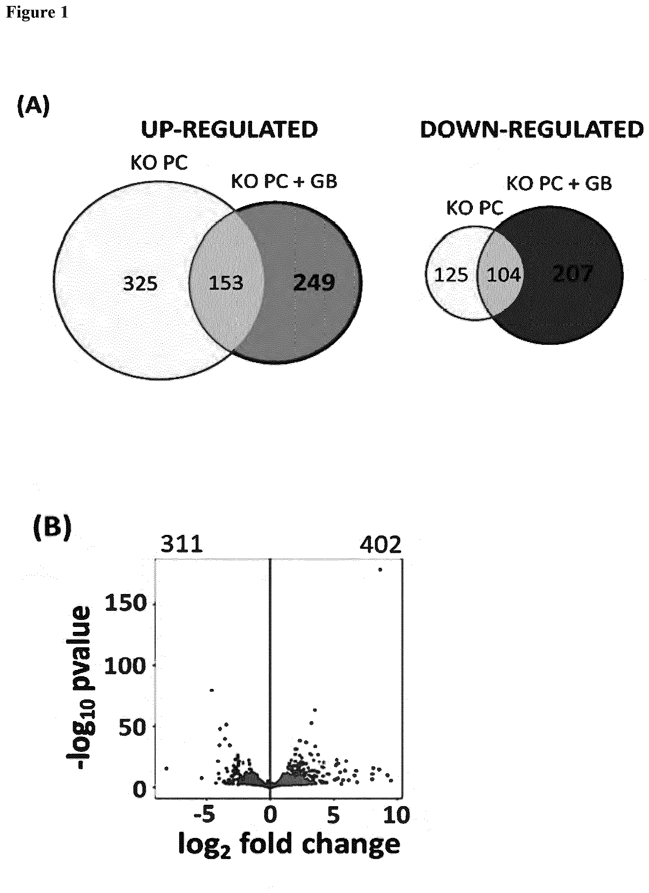

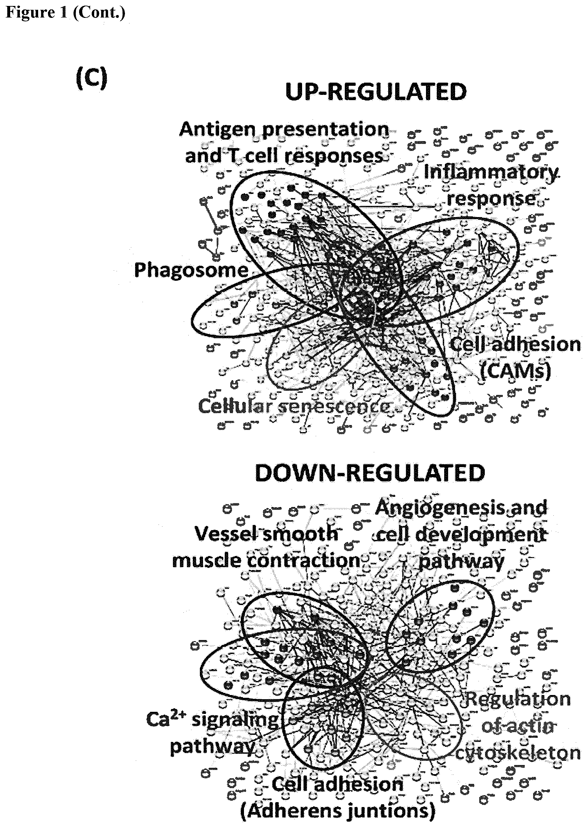

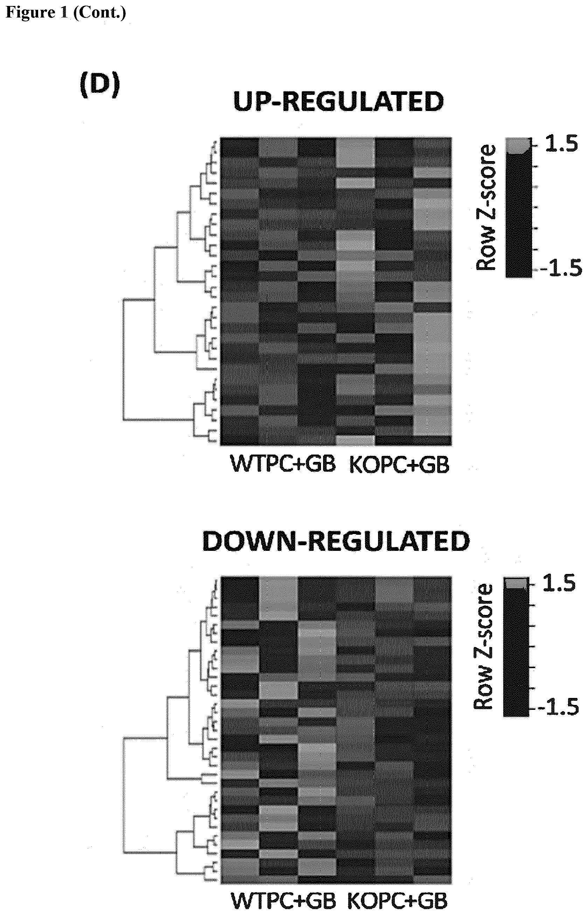

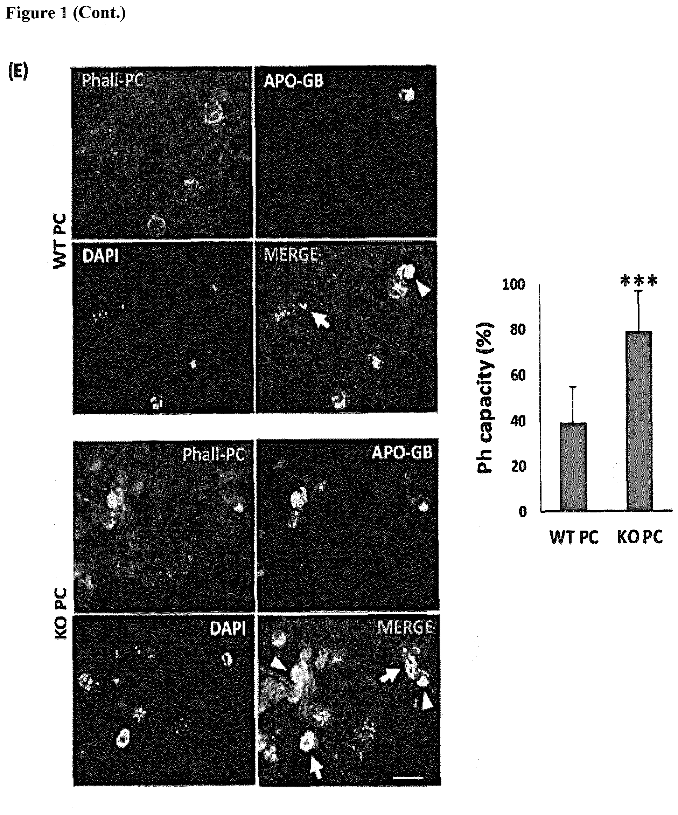

**Figure 1****.** CMA-dependent differential expression of genes (DEGs) and main pathways affected. **(A)** Venn diagram of significantly DEGs "up-regulated" or "down-regulated" in KO PC compared to WT PC in the presence or the absence of GB (FDR<0.01). **(B)** Volcano plot of all significant DEGs in KO PC +GB compared to WT PC+GB. In red are shown the 402 up-regulated genes and in blue, the 311 down-regulated genes. The default set of threshold was FDR<0.01. **(C)** Network visualization of Gene Ontology enrichment of proteins of the main selected affected up-regulated (anti-tumoral; above) or down-regulated (pro-tumoral; below) pathways from CMA-dependent DEGs. Major clusters are circled, and node size indicates the number of proteins per node. **(D)** Heatmap of up-regulated CMA-dependent DEGs (above) and down-regulated CMA-dependent DEGs (below) corresponding to the main affected pathways in all the three samples of each condition (WT+GB 1, WT+GB 2, WT+GB 3, KO+GB 1, KO+GB 2, KO+GB 3). Red boxes represent down-regulated genes, and green boxes represent up-regulated genes. The value of expression intensity is based on the gene expression level analysis. **(E)** Phagocytic capacity (Ph capacity) of KO PC (below) versus WT PC (above) against GB cells. Images show representative images of PC, visualized with phalloidin (Phall-PC; green) displaying phagocytic activity by engulfing of pyknotic nuclei (with the DNA dye DAPI, white; arrows) and/or cytoplasmic inclusions (arrowheads) of apoptotic GB stained with Dil and phalloidin (APO-GB; yellow) (Scale bars: 20 µm). Data represent mean ± SD obtained from images of at least 5 phagocytosis experiments using apoptotic U373 and U87 GB cell lines independently. Scale bar: 20 µm; ***p<0.005.

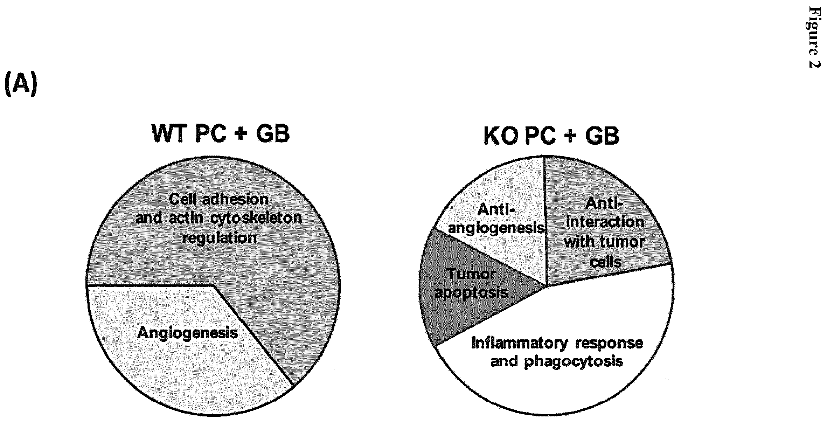

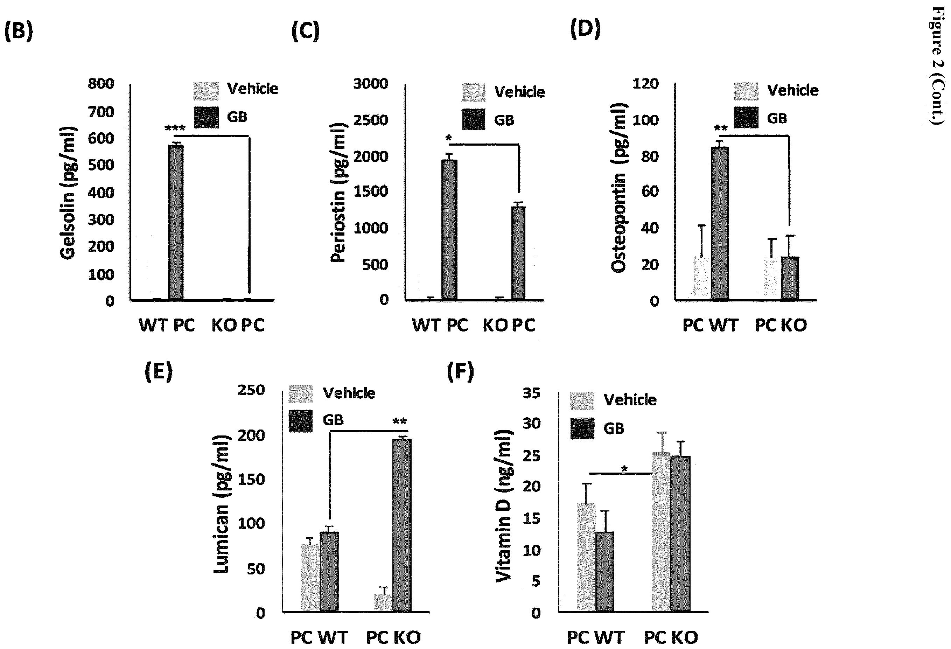

**Figure 2****.** GB Prognostic marker analysis. **(A)** Mass spectrometry general diagram shows the analysis of functional groups associated to pro-tumor and anti-tumor mechanisms, representing those proteins differently identified in the secretome of pro-tumor WT PC+GB and anti-tumor KO PC+GB co-cultures, respectively. **(B)** to **(F)** Validation by ELISAs of the identified molecules that are associated with the pro-tumor or anti-tumor immune response. Those can be useful for GB prognosis in future studies with GB human biopsies. Concentrations of mouse gelsolin, periostin, osteopontin, lumican, and vitamin D produced by WT PC and KO PC in the absence (vehicle) or the presence of GB for 72 hours are shown. *p<0.05, **p<0.01.

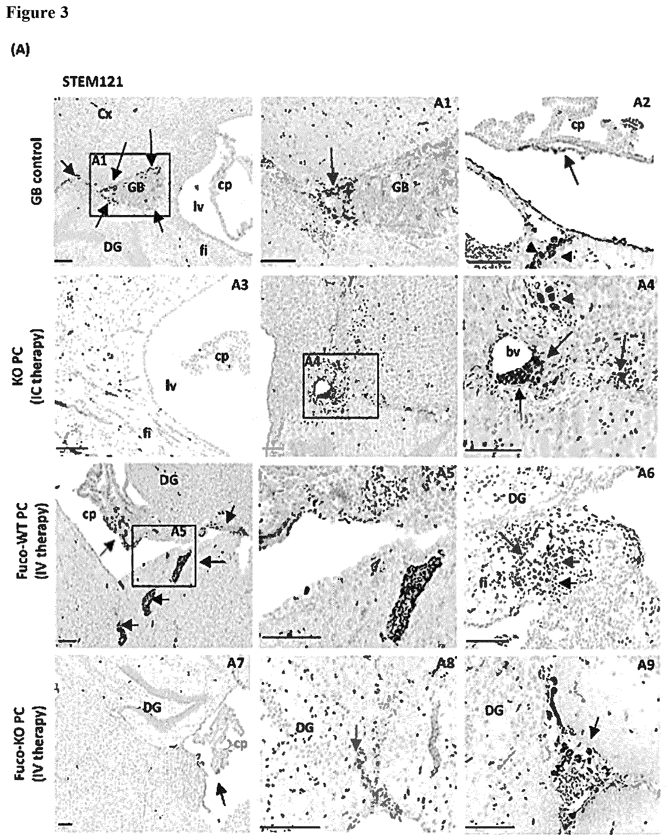

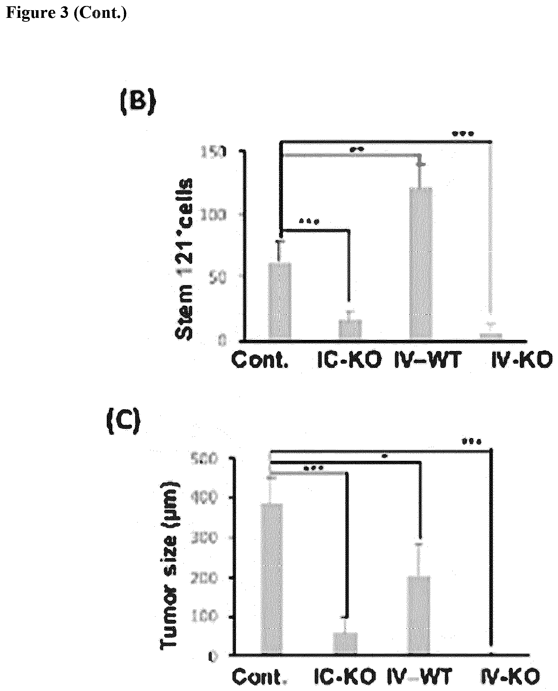

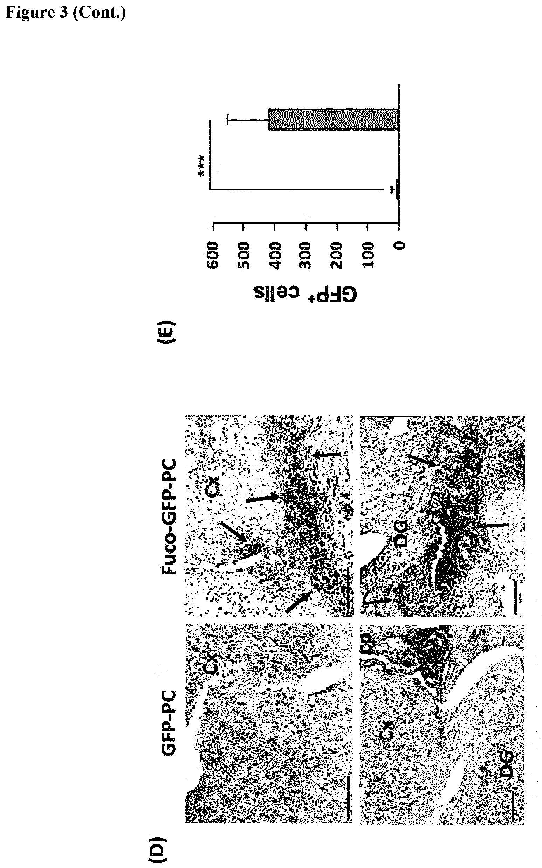

**Figure 3**. **(A)** GB proliferation in xenografts from mice treated with different strategies (IC therapy: Intracranial therapy with KO PC; IV therapy: Intravenous therapy with exofucosylated (Fuco) WT or KO PC after 1 month of GB tumor proliferation (GB control). GB cells were stained with STEM121 antibody. Arrows show tumor formation (GB control) around perivascular areas (arrow in A1) of the brain cortex (Cx) and infiltration of tumor cells in choroid plexus (arrow in A2) and subpial vessels (arrowheads in A2). The KO PC IC therapy shows a representative image (A3) of the grafted brain areas without rest of tumor and just some few tumor cells close to a blood vessel (bv; arrows in A4), graft trajectory and subpial meninges (arrowheads in A4). Representative images of meningeal tumor infiltration in a Fuco-WT PC IV therapy, tumor cell proliferation in choroid plexus (cp) and the brain parenchyma (arrows A5), tumor cells populated the most caudal part of the hypocampus and dentate gyrus (DG) and fimbria (fi; A5,6). Some rest of tumor cells of a previous tumor outside of the brain parenchyma were hardly detected in choroid plexus, perivascular and subpial regions of DG with the Fuco-KO PC IV therapy (arrows in A7,9) **(A)** (Scale bars: 100 µm). Shown images are representative of both U373 and U87, using U87 GB line. **(B)** Relative quantification of tumor cells related to the number of immunopositive stem 121 cells **(C)** Morphometric measurement reveals the average of tumor size in GB control mice (6 tumors / 5 grafted mice), GB mice intracranially treated with KO PC (IC-KO; 3 tumors/ 5 grafted mice), GB mice treated with Fuco-WT (IV-WT; 13 tumors/ 5 grafted mice or KO PC (IV-KO; 0 tumors/ 5 grafted mice). **(D)** Grafts of exofucosylated GFP-PC that were injected intravenously in the mice grafted with GB cells previously, were compared to those from the GB mouse model just treated with GFP-PC and stained with an anti-GFP antibody (Scale bars: 100 µm). **(E)** Quantification of the number of fucosylated PC (Fuco-GFP-PC) that reach tumor areas after therapy compared to control (GFP-PC) and related to the number of GFP immunopositive cells. All results are mean+SD from at least three different experiments using both U373 and U87 GB lines, independently. *p<0.05, **p<0.01, ***p<0.001.

### Detailed description of the invention

The present invention is illustrated by means of the Examples set below without the intention of limiting its scope of protection.

### Example 1. Material and methods

### Example 1.1. Mice

Eight to twelve-week-old WT C57BL/6, C57Bl/6-Tg (ACTB-EGFP)1Osb/J (Charles River laboratory) mice were maintained in pathogen-free conditions in the animal facilities of the University of Murcia and Biomedical Research Institute of Murcia Virgen de la Arrixaca. All animal procedures were approved and performed according to the guidelines set by the University of Murcia Institutional Animal Care and Use Committee.

### Example 1.2. Cell Culture

Primary brain PC from mice were isolated and co-cultured with GB cells at a ratio 1:1 for 72 h. PC with impaired CMA (KO PC), were isolated from brains of Lamp2a-/- mice. Human GB cell lines U373-MG and U87 were purchased from European Collection for Authenticated Cell Cultures. Cell culture media obtained from 72 h co-cultures of GB and PC was concentrated using Amicon Ultra centrifugal filters 10k (Millipore) and used 10 times diluted. Dil labeling solution (Invitrogen) and GFP-expressing PC were used for cell tracking.

### Example 1.3. RNA sequencing and differential expression analysis

For RNA-seq, total RNA from WT PC, KO PC and GB, single and co-cultured PC-GB, was extracted with the purification RNA RNeasy Mini Kit following manufacturer instructions and treated with DNase I (Qiagen). Equal amounts of purified total RNA from 3-4 experiments of each one was pooled in each sample. DNA libraries for small RNAs and mRNAs were processed and sequenced by the CRG core genomics facility (Barcelona, Spain) using a HiSeq-2500 apparatus (Illumina, service provided by Fasteris S.L.) according to the manufacturer's instructions. For the quality control, read alignment, obtaining metrics for gene expression, please see supplementary text. Differentially expressed genes (DEGs) between GB conditioned PC (GB-WT PC) and CMA-deficient PC with GB (GB-KO PC) were detected using DESeq2 v1.18.1 package in R computing platform v3.4.4. DEGs were computed using batch correction in the formula design (design= ∼condition+ sample_batch). Genes with FDR Adj. *p* < 0.01 were considered significantly differentially expressed.

### Example 1.4. Heatmap, functional annotation and pathway analyses

A heatmap was generated to visualize the expression values of the interest up-regulated genes and another for the visualization of the down-regulated ones in GB-KO PC vs GB-WT PC with FDR < 0.01. To generate the heatmap, the heatmap.2 function of the R (R Core Team, 2021) g plots package was used. Network visualization of Gene Ontology enrichment of proteins of the main affected up-regulated or down-regulated pathways was performed by STRING v11.5 functional protein association networks. Major clusters are circled, and node size indicates the number of proteins per node.

### Example 1.5. In vitro phagocytosis assay

Briefly, WT PC and KO PC were allowed to rest and settle for at least 48 h before phagocytosis experiments in 24-well plates. GB cell lines were previously labeled with the cell tracker Dil and treated with 60 µM of staurosporine (Cayman Chemical) for 48 h to induce apoptosis. Only the floating dead-cell fraction was collected from the supernatant and added to the PC cultures in a proportion of 1:1. Apoptotic cells were visualized and quantified by trypan blue exclusion. Because cell membrane integrity is still maintained in early induced apoptotic cells, cells not labeled with trypan blue were considered apoptotic. After 2 h, cells were fixed with 4% paraformaldehyde in PBS after washing away apoptotic non-adhered cells with media. Remanent apoptotic cells and PC were stained with AlexaFluor 488 Phalloidin (Invitrogen) to detect F-actin cytoskeleton. Images were acquired with a Delta Vision RT (Applied Precision) restoration microscope coupled to a Coolsnap HQ camera (Photometrics), with a 60x/1.42 Plan Apo or 100x/1.40 Uplan Apo objectives. The percentage of PC with phagocytic pouches (Ph capacity) was counted. Morphometric measurements and quantification of cells were performed using ImageJ (NIH, USA) and Adobe Photoshop (Adobe, Inc) software. Pictures for illustrations and quantitative analysis were uploaded from direct microscopic images and were not manipulated in subsequent steps of figures preparation, except for framing and scaling.

### Example 1.6. Secretome analysis

Concentrated cell culture media from WT PC or KO PC, cultured alone or co-cultured with GB, control GB and control cell culture media were depleted from major serum proteins through spin-column chromatography *BluePrep* Major Serum Protein Removal Kit (SERVA) and quantified by the IMIB Virgen de la Arrixaca Proteomics facility. Subsequently, the proteins of the supernatants were digested with trypsin (see supplementary text), identified by means of HPLC-MS/MS analysis (see supplementary text) and validated using auto thresholds by the Proteomics facility of the University of Murcia (See supplementary text). The separation and analysis of the tryptic digests of the samples were performed with a HPLC/MS system consisting of an Agilent 1290 Infinity II Series HPLC (Agilent Technologies) equipped with an Automated Multisampler module and a High Speed Binary Pump, and connected to an Agilent 6550 Q-TOF Mass Spectrometer (Agilent Technologies) using an Agilent Jet Stream Dual electrospray (AJS-Dual ESI) interface. Experimental parameters for HPLC and Q-TOF were set in MassHunter Workstation Data Acquisition software (Agilent Technologies, Rev. B.08.00). Finally, the differential expression of secreted proteins in each experimental condition was analyzed by the IMIB Virgen de la Arrixaca bioinformatics service. The proteins in the culture medium from serum and from GB cultures were subtracted from the KOGB and WTGB averages and the ratio of the averages was determined. The default set of threshold was log2FoldChange ≥ 1,25.

### Example 1.7. ELISA

PC (5×10⁴) were co-cultured with GB cells at 1:1 ratio in 96-well plates for 72 h. Mouse gelsolin, periostin (Wuhan Fine Biotech co.), osteopontin (Abclonal), lumican and 25-HO Vitamin D (Arigo Biolaboratories) levels secreted by PC in the media were measured by sandwich ELISA with specific anti-mouse antibodies following the manufacturer's recommendations.

### Example 1.8. Pericyte exofucosylation

Murine pericytes were modified by enzymatic exofucosylation. Briefly, cells were resuspended at 2x10⁷ cells/ml in fucosyltransferase VII (FTVII) reaction buffer composed of Hanks Balanced Salt Solution (HBSS, Gibco) containing 30 µg/ml FTVII (R&D Systems), 20 mM HEPES (Thermo Fisher Scientific), 0.1% human serum albumin (Merck Millipore) and 1 mM guanosine 5'-diphospho-β-L-fucose sodium salt (GDP-fucose, Sigma Aldrich), and incubated for 60 min at 37°C and 5% CO₂. Unmodified control pericytes were treated only with GDP-fucose (w/o FTVII) in the same conditions as above. Cell viability after exofucosylation was assessed by trypan blue exclusion (usually 95% live cells). Efficacy of exofucosylation was evaluated by analysis of HECA452 antibody (BD Biosciences) staining and calcium dependent mouse E-human IgG chimera (R&D Systems) binding by flow cytometry.

### Example 1.9. Xenografts and therapeutic strategies

Cell pellets from human GB cells (5×10⁶ cells) were xenografted into n=40 C57BL/6 mice brains. Xenografts were performed intracranially in the brain hyppocampus using an exterotaxic surgery. Three weeks postgrafting, mice were treated with different therapeutical strategies to compare to those none treated. Five mice were intracraneally grafted with KO PC (intracranial therapy, IC therapy); five mice were injected intravenously with exofucosylated WT or KO PC (Intravenous therapy, IV therapy); and five mice were injected intravenously with unmodified or exofucosylated GFP-PC. Four weeks after therapies, mice were sacrificed, and brains were fixed in 4% buffered formalin (Panreac Quimica). All animal procedures described were repeated three times using different glioblastoma cell lines independently (U-87 and U-373).

### Example 1.10. Immunohistochemistry

Brains were paraffin embedded and processed by the Pathology facility (IMIB Virgen de la Arrixaca). Three-micrometer thick serial sections were obtained from paraffin embedded samples using an automatic rotary microtome (Thermo Scientific). For colorimetric immunolabeling, sections were incubated overnight at 4°C with mouse anti-human STEM121 (Cellartis), rabbit anti-GFP (Abcam), primary antibodies. Sections were finally incubated with the corresponding 3-3'Diaminobencidine (DAB) secondary antibodies (Vector Labs) and hematoxylin counterstained. Positive immunoreaction was identified as a dark-brown precipitated. An automatic digital slide scanner (Pannoramic MIDI II-3DHistech) and Quantitative Pathology & Bioimage Analysis Qupath-0.2.3 software were used for analysis of histological sections and acquisition of images.

### Example 1.1. Statistical Analysis

Differences between groups were analyzed by one-way ANOVA followed by Tukey-Kramer posttest. Comparisons between data pairs were analyzed using a t test. Statistical significance was defined as P < 0.05.

### Example 2. Results

### Example 2.1. Transcriptomic profiling in CMA-deficient PC in response to GB reveals specific target functions useful in therapy

To reveal the gene pathways affected by GB-induced CMA in PC, we performed RNAseq studies to compare the differentially expressed genes (DEGs) between LAMP2A KO PC and WT PC, both in absence and in presence of GB. A total of 707 DEGs were detected between KO PC compared to WT PC in absence of GB, of which 478 genes were up-regulated (higher expression in deficient CMA PC) and 229 genes were down-regulated **(****Figure 1A****).** On the other hand, a total of 713 DEGs were also identified to be dependent of CMA but also of GB, of which 402 genes were up-regulated in KO PC + GB and 311 genes were down-regulated **(****Figures 1A** and **B**). But most importantly, a total of 456 DEGs from those genes, were detected to not-overlap with just CMA-dependent DEGs between KO PC and WT PC. 249 genes were up-regulated in KO PC + GB and 207 genes were down-regulated (grey dark circles in **Figure 1A**).

DEGs were analyzed by the Gene Ontology enrichment to determine the affected biological pathways. Kyoto Encyclopedia of Genes and Genomes (KEGG) pathway enrichment analysis from CMA-dependent DEGs revealed several gene expression pathways up- or down-regulated in CMA-deficient PC and affected by GB-induced CMA **(****Figure 1C****).** Moreover, the up-regulated and down-regulated genes of the selected CMA-dependent pathways **(****Figure 1C****)** were corroborated by a heatmap **(****Figure 1D**).

In agreement with our previous findings, the main affected up-regulated pathways in KO PC in presence of GB were related to immune and inflammatory responses, and other anti-tumor cell functions such as cell-adhesion **(****Figure 1C****,** above), which is altered in CMA-deficient PC and prevents stable interaction with GB cells.

In addition, the network visualization of these affected CMA-dependent up-regulated pathways in KO PC showed large overlap between them **(****Figure 1C****,** above). On the contrary, the main affected down-regulated pathways such as angiogenesis, cell adhesion, regulation of actin cytoskeleton and others (**Figure 1C****,** below) were related to the pro-tumoral functions observed in PC with GB-induced CMA. Interestingly, the network visualization of the main affected CMA-dependent down-regulated pathways showed also an overlap between them with the exception of the angiogenesis and regulation of actin cytoskeleton pathways, which appeared independent from the others (**Figure 1C****,** below).

Importantly, one of the up-regulated CMA-dependent pathways that was identified in KO PC was the phagosome pathway, which suggested an increase in the PC phagocytic activity as an anti-tumor function of KO PC. To validate phagocytic activity, we compared the phagocytic capacity of WT and KO PC in co-cultures with apoptotic GB. Apoptotic cells, which have been previously characterized to express other apoptosis markers such as activated caspase 3 and fractin, were defined as pyknotic/karyorrhectic nuclei labelled with the DNA dye DAPI. The PC phagocytic capacity (Ph capacity, i.e., the proportion of pericytes with one or more phagocytic pouches, each containing one apoptotic cell) was significantly increased in KO PC **(Figure IE).** Taking together all the previous results, the CMA ablation in PC in response to GB cells showed an immunogenic phenotype able to phagocyte GB cells and a key strategy to develop future therapies against GB cancer.

### Example 2.2. Differential expression of protein secretion from co-cultures of GB with CMA-deficient PC reveals new prognosis markers for tumor progression

Abnormal upregulation of CMA is a mechanism by which GB cells elicit the immunosuppressive function of PC and stabilize GB-PC interactions necessary for tumor cell survival. CMA-deficient PC co-cultured with GB cells result in the secretion of proteins that reduce tumor cell survival through prevention of PC-GB interactions, and disruption of the pre-established ones. In order to elucidate the specific contribution of CMA-dependent secretome to tumor cell survival, we performed comparative proteomics of proteins secreted in GB-conditioned PC versus CMA deficient PC in presence of GB. As a result, several secreted proteins associated to pro-tumoral or anti-tumoral functions were identified in each condition, respectively **(****Figure 2A****).**

### Pro-tumoral immune proteins

We identified several protein fractions enriched in the secretome of GB-conditioned PC. According to Biological Processes of Gene Ontology, they were proteins associated to cell adhesion, actin cytoskeleton regulation, and angiogenesis functions (**Figure 2A****,** left). All of them were related to pro-tumoral functions of GB-conditioned PC and in concordance with the affected cell pathways that were detected **(****Figure 1****).** Some of these interesting proteins that have been related to the pro-tumor immune response, such as gelsolin, periostin or osteopontin, were exclusively detected in the secretome of GB-conditioned PC, and not in the other conditions. As these interesting proteins might be good markers for GB prognosis dependent on CMA in PC, we experimentally confirmed the presence of these proteins and their mouse origin in the secretome of GB-conditioned PC. Interestingly, our results revealed that all proteins, were dependent on GB-induced CMA in PC **(****Figure 2B-D**).

### Anti-tumoral proteins

On the contrary, different protein fractions were enriched in the secretome of CMA deficient PC in presence of GB (**Figure 2A****,** right). According to Biological Processes of Gene Ontology, they were proteins associated to inflammatory response, phagocytic activity, anti-angiogenic functions, and anti-interaction proteins of tumor cells. As all of them are related to anti-tumoral functions of KO PC in presence of GB and in concordance with the affected pathways detected **(****Figure 1****),** we confirmed the levels of some of these interesting proteins related to the anti-tumor immune response for good GB prognosis **(****Figure 2** **E, F).** Lumican, a matrix protein which have an anti-tumor role inhibiting or even reversing several metastatic features in cancer cells, was produced in all experimental conditions, but mainly by KO PC in presence of GB as CMA dependent anti-tumor protein **(****Figure 2E****).** Instead, the secretion levels of vitamin D, as an anti-tumor molecule in PC, were CMA-dependent, but not GB-dependent since its secretion level were higher in KO PC, regardless of whether GB is present or not **(****Figure 2F****).** Thus, this last finding was not only useful for the prognosis of GB progression dependent of CMA, but even as a fact to subsequently develop therapeutic strategies against GB using CMA-deficient PC.

### Example 2.3. Exofucosylated PC with deficient CMA were efficient to reach the tumoral niche and eliminate the tumor cells by intravenous therapy

We have seen previously that GB-induced CMA in PC assists tumor growth *in vivo* through GB-PC interactions and failed anti-tumor T cell responses. The lack of CMA in PC with GB prevents PC-GB interactions, the secretion of proteins that reduce tumor cell survival and the acquisition of an immunosuppressive function in PC following tumor interaction. To determine if CMA ablation in PC would still allow these cells to reach the tumoral niche and eliminate tumor cells, and therefore, a useful approach for the settlement of future therapies, we first analyzed the tumor growth of our xenograft mouse model of GB after being treated with unmodified or exofucosylated PC, a bioengineering strategy that has been previously shown to enforce expression of HCELL, a CD44 glycovariant that is a potent E-selectin ligand, and to increase PC colonization within lesional sites after intravenous administration **(****Figure 3**, **C**). GB control mice showed brain infiltration of tumor cells in choroid plexus and subpial vessels, and tumor proliferation in brain cortex around perivascular areas. GB control mice that were grafted with KO PC, showed, however, just some few tumor cells close to blood vessels with no other rest of tumor, after one month of the therapy (intracranial therapy: IC; **Figure 3A**, **B**). Higher GB progression in the brain parenchyma and tumor proliferation and infiltration were observed in the GB mouse model treated with exofucosylated WT PC (Fuco-WT PC; intravenous therapy: IV) compared to control mice and the other strategies. In contrast, cells of a previously engrafted tumor were hardly detected outside of the brain parenchyma in the GB mouse model after being treated with exofucosylated KO PC (Fuco-KO PC; IV; **Figure 3A****, B**). Although the difference in results after therapy with Fuco-WT/KO PC was obvious, we wanted to demonstrate that PC were able to reach the tumor niche, going through the blood brain barrier (BBB) to eliminate tumor cells **(****Figure 3C****)**. For this, the GB model was intravenously injected with Fuco-GFP-PC and compared to those injected with control unmodified GFP-PC. A Fuco-GFP-PC accumulation in the affected areas was detected after 1 week of treatment **(****Figure 3C**, **D**).

Flow cytometry analyses of CD4⁺ T cells from central draining lymph nodes of the GB mice treated with different strategies showed that either intracranial or intravenous therapy with KO PC seem to be effective to activate the anti-tumor T cell responses. After therapy with KO PC, T cells presented significant higher levels of PD-1 and CTLA-4, two inhibitory T cell receptors that are present in activated T cells, exhausted T cells and some subsets of memory T cells. Whereas the levels of the T regulatory cells (Tregs) transcription factor FoxP3 was not affected in any of the therapies.

In agreement with previous results on the CMA-dependent phagocytic capacity in PC, we also found that depending on the therapy type different phagocytic cell populations are contributing to the tumor clearance in the anti-tumor innate response. The microglia activation marker Iba-1, also expressed in activated PC and macrophages was found expressed in grafts of the GB control mouse model, showing gliosis accumulation in the tumor and peritumoral areas. Grafts from mice treated with the KO PC IC therapy showed a significant accumulation of activated microglia in previous tumor areas and some cell debris in perivascular areas where there were still some tumor cells. Excitingly, the Fuco-KO PC IV therapy showed great immunoreaction for Iba-1 in microglia, perivascular cells and infiltrated blood cells along perivascular areas and close to previous tumorigenesis that was eliminated. However, Iba-1 immunopositive cell debris were hardly observed around tumor cell areas after the Fuco-WT PC IV therapy. Interestingly, the macrophage activation marker CD68, also expressed in some cases in activated PC and microglia, was highly expressed just in grafts of the mice treated with the KO PC intracranial therapy.

## Claims

1. Isolated pericyte cell, or secretome derived thereof, wherein the pericyte is **characterized in that** the chaperone-mediated autophagy (CMA) is inhibited or impaired.

2. Isolated pericyte cell, or secretome derived thereof, according to claim 1, wherein the pericyte is **characterized in that** the expression of the gene LAMP2A is inhibited or the gene LAMP2A is deleted.

3. Isolated pericyte cell, according to any of the claims 1 or 2, derived from adipose tissue or any vascular-stromal compartment of microvascularized tissues comprising brain, breast, kidney or liver.

4. Substantially pure population of pericytes according to any of the claims 1 to 3, or cell suspension comprising pericytes according to any of the claims 1 to 3, wherein the pericytes are at least 80% of the total cell population or suspension.

5. Isolated pericyte cell according to any if the claims 1 to 3, or substantially pure population of pericytes, or cell suspension comprising pericytes, according to claim 4, for use as a medicament.

6. Isolated pericyte cell according to any if the claims 1 to 3, or substantially pure population of pericytes, or cell suspension comprising pericytes, according to claim 4, for use, according to claim 5, in the treatment of cancer.

7. Isolated pericyte cell according to any if the claims 1 to 3, or substantially pure population of pericytes, or cell suspension comprising pericytes, according to claim 4, for use, according to claim 6, in the treatment of glioblastoma.

8. Isolated pericyte cell according to any if the claims 1 to 3, or substantially pure population of pericytes, or cell suspension comprising pericytes, according to claim 4, for use, according to claims 6 or 7, wherein the CMA of the pericyte is inhibited or impaired before being administered to the patient.

9. Pharmaceutical composition comprising the isolated pericyte cell of any of the claims 1 to 3, or a substantially pure population of pericytes, or a cell suspension comprising pericytes, according to claim 4 and, optionally, pharmaceutically acceptable carriers or excipients.

10. *In vitro* method for obtaining a pericyte cell, or cell suspension comprising pericytes for use as a medicament, wherein the method comprises inhibiting CMA in the pericyte, preferably by deleting the gene LAMP2A or inhibiting its expression.

11. *In vitro* method for obtaining a pericyte cell, or a cell suspension comprising pericytes, according to claim 10, for use in the treatment of cancer, preferably glioblastoma, wherein the method comprises inhibiting CMA in the pericyte, preferably by deleting the gene LAMP2A or inhibiting its expression.

12. *In vitro* method according to any of the claims 10 or 11 wherein the gene LAMP2A is deleted using a gene editing technique selected from: CRISPR-Cas Gene Editing Restriction Enzymes, Zinc Finger Nucleases, TALENs Gene Editing or RNA interference.

13. *In vitro* method according to any of the claims 10 to 12 wherein the CMA, preferably the expression of the gene LAMP2A, is inhibited using pharmaceutical or biologic inhibitors.

14. *In vitro* use of the isolated pericyte, or the secretome derived thereof, of any of the claims 1 to 3, or the substantially pure population of pericytes, cell suspension comprising pericytes, according to claims 4, for obtaining biomarkers to be used in the diagnosis of cancer, preferably glioblastoma.

15. *In vitro* method for the diagnosis and/or prognosis of cancer, preferably for the diagnosis of glioblastoma, which comprises assessing, in the secretome of a pericyte according to any of the claims 1 to 3 isolated from a subject, the level of at least a biomarker selected from the group comprising: lumican, vitamin D, gelsolin, periostin and/or osteopontin, wherein:
a. The identification of a higher level of lumican and/or vitamin D as compared with a pre-established threshold level determined in healthy control subjects, is an indication that the patient suffering from cancer, preferably from glioblastoma, has a good prognosis, and/or a good chance of therapy success.
b. The identification of a higher level of gelsolin, periostin and osteopontin as compared with a pre-established threshold level determined in healthy control subjects, is an indication that the patient suffering from cancer, preferably from glioblastoma, has poor prognosis.
